# EUROPEAN PATENT SPECIFICATION

(11) **EP 2 399 576 B1**
(45) Date of publication and mention of the grant of the patent: **03.01.2018**
(21) Application number: 11170675.0
(22) Date of filing: 21.06.2011
(51) Int. Cl.: A61K 31/185, A61K 31/403, A61K 33/30, A61P 1/02, A61P 31/02, A61P 31/04, A61K 8/27, A61K 8/46, A61Q 11/00, A61Q 17/00

(54) **Use of antibacterial compounds for the oral cavity hygiene**
Verwendung von antibakteriellen Verbindungen zur Pflege der Mundhöhle
Utilisation de composés antibactériens pour l'hygiène de la cavité buccale

(30) Priority: 25.06.2010 IT MI20101153
(43) Date of publication of application: 28.12.2011
(73) Proprietor: Farmaceutici Dott. Ciccarelli S.p.A., 20138 Milano (IT)
(72) Inventor: Pasetti, Marco, 20121 MILANO (IT); Cerini, Roberto, 35020 PADOVA (IT); Antonini, Antonella, 43100 PARMA (IT); Fedele, Maria Rosaria, 20137 MILANO (IT)
(74) Representative: Sama, Daniele

(56) References cited:
- EP-A1- 1 050 294
- EP-A2- 1 074 247
- WO-A1-96/25913
- US-A1- 2006 099 152

## Description

The present invention relates to the use of particular antibacterial compounds for the oral cavity hygiene.

More specifically the present invention relates to the use in formulations for the hygiene of the oral cavity of zinc compounds having high antibacterial activity and high activity also on yeasts such as Candida Albicans, combined with improved formulation stability, without requiring the addition of surfactants as essential components.

It is known to use zinc compounds in formulations for the oral hygiene, as well as, more generally, in the cosmetic field. See for example WO 96/25,913 which describes compositions having an antimicrobial activity containing monophase zinc hydroxycarbonate and a process for its preparation. The compositions can be in the form of toilette soaps, products for the skin and for hairs and products for the oral hygiene such as toothpastes, powders and dental creams. In the background of this patent application it is stated that it is known that divalent zinc cations can give antimicrobial activity, if they are freely available in compositions such as soaps, cosmetics or cosmetic or dental formulations, they contribute to the health care properties. However free zinc ions have the drawback to affect the organoleptic characteristics, such as the colour and/or the taste of cosmetic products or for the hygiene of the oral cavity and can reduce the stability of these products. Due to these grounds in relation to the structure of the zinc hydroxycarbonate, wherein zinc is present in the form of covalent stable complexes, free zinc cations are not made available in the compositions. Therefore the stability of these compositions is improved. According to this patent application, zinc hydroxycarbonate, which is not soluble in water but soluble in the presence of an acid, is deemed to release divalent zinc ions acting on microbes generating acids, such as lactic and butyric acid. The antimicrobial action of the zinc ions is increased by the presence of surfactants, such as soaps or synthetic detergents, which confer synergistic action.

In this reference it is also stated that the synergistic action is obtained also by using detergents or anti-dandruff compounds such as zinc pyrithione for shampoo formulations.

Synergistic action of the hydroxycarbonate zinc takes place also in compositions for the skin and talcum powders.

Neutralization of organic acids generated on the skin, such as butyric acid, that is responsible for the bad skin odour, can be obtained with zinc hydroxycarbonate, as zinc ions act on the skin microflora.

Patent application WO 98/17,195 relates to compositions comprising a zinc compound wherein the cation is freely available and at least one compound increasing or stabilizing the oxidoreduction potential of the oral cavity. In the background of this patent application it is stated that in the oral cavity the bacteria responsible for the formation of the degradation compounds causing halitosis and periodontitis are the gram-negative ones, in particular the anaerobes. According to this patent application it is possible to inhibit their appearance by contrasting the capacity of these bacteria to lower the oxidoreduction (Eₕ₎ potential of the oral cavity and at the same time they increase the oxidoreduction potential. According to this reference the presence of the zinc species in the form of cations is important since it inhibits the capacity to lower Eₕ by the bacterial flora. The compositions contain zinc compounds associated with hydrogen peroxide and compounds containing chloride ions. Instead of hydrogen peroxide, an oxyhalogenated compound can be used, for example chlorites as reported in the application, or methylene blue. The hydrogen peroxide concentration ranges from 0.1 to 3% by weight. In the formulation this compound is kept separated from the compound containing zinc since this metal negatively affects the stability of the composition. The preferred pH is comprised between 3 and 6.

The drawback of the compositions of this document is that hydrogen peroxide and oxyhalogenated compounds are used which are aggressive on the oral cavity mucosa, especially in case of prolonged treatments, as usually requested for halitosis and periodontitis. Besides, the pH of these compositions can be also acid, thus create an aggressive environment for the tooth enamel. Furthermore the formulation requires separate compartments.

Patent application WO 97/26,855 relates to mouthwashes for preventing or eliminating the halitosis and for reducing the microorganisms responsible for the formation of dental plaque and tooth loss.

The composition contains: (a) an effective amount for antimicrobial activity of thymol and one or more essential oils, (b) ethanol up to about 30% v/v, (c) from 0.1% to 8% by weight of a peroxide, (d) an amount of surfactant for solubilizing the essential oils, (e) water. It is reported that the composition can be added with zinc chloride or other zinc salts, such as for example zinc gluconate, zinc sulphate, etc., as topical astringent, in an amount from 0.0025% to 0.200% w/v.

This composition shows the same drawbacks of the previous application WO98/17,195.

USP 5,310,546 concerns mouthwashes for the oral cavity hygiene containing, as % weight/volume, from about 0.25% to about 0.65% of hydrogen peroxide, from 0.005% to 0.1% of zinc chloride, at least 0.012% of sodium citrate, at least 0.03% of sodium lauryl sulphate, at least 0.006% of citric acid, and ethanol in an amount lower than 3%. The citric acid has the function to maintain the pH in a range from 3.5 to 4.5.

The drawback of these compositions is the same described for the compositions of the previous patent applications WO98/17,195 and WO97/26,855 wherein hydrogen peroxide is used.

Patent application EP 1,074,247 describes detergent compositions comprising zinc alkylsulphates or zinc alkylpolyethoxysulphates as surfactants and preservatives. The compositions have anti-dandruff, sebonormalizing and deodorant properties.

It is also known in the prior art that the oral cavity is colonized by a composite bacterial flora which must be controlled and removed to avoid the formation of the bacterial plaque, which causes tooth and gum pathologies and diseases, such as tooth decay, tartar, gingivitis, paradontal diseases.

The prior art compositions for controlling and removing the bacterial flora at present available on the market contain various compounds, among them Triclosan, (INCI name) 5-chloro-2-(2,4-dichlorophenoxy)phenol is the most used. This compound has the advantage to be very active against oral cavity bacteria. However recently this compound is under investigation for its toxicological properties (safety) by the European Toxicology Commettee (Scientific Committee on Consumer Product - SCCP - Opinion on Triclosan - COLIPA n° P32 1 January 2009). In particular, it appears that this compound causes bacterial resistance. Bacterial resistance is the ability of some microbial species to survive, or to reproduce, in the presence of concentrations of antimicrobial agents normally sufficient to inhibit or kill microorganisms of the same species.

Therefore there is the need to have available substitutes of Triclosan in the formulations for the oral cavity hygiene, having the following combination of properties:
- equal or comparable activity on bacterial strains of the oral cavity, in particular Streptococcus mutans, Streptococcus agalactiae, Staphylococcus aureus, Actinomycetes, Candida albicans (yeast),
- use safety,
- compatibility (drop-in) comparable to that of Triclosan with the conventional raw materials used in these formulations and contemporaneously without the use of any surfactant.

It has unexpectedly and surprisingly been found by the Applicant antibacterial compounds which can be used as drop-in of Triclosan in formulations for the oral cavity hygiene.

It is an object of the present invention the use of zinc compounds having formula:

[R-(OCH₂CH₂)ₙ-OSO₃⁻] • 1/2 Zn²⁺ (I)

wherein R is a linear or branched, saturated or unsaturated aliphatic chain, having from 6 to 20 carbon atoms, n is an integer from zero to 20, for the preparation of formulations as antibacterial agents of the oral cavity, as defined in the claims. Preferably in the compounds of formula (I) n=0.

Mixtures of the compounds of formula (I) can also be used.

Preferably the aliphatic chain is C₈-C₁₈ and is linear. More preferably the aliphatic chain is linear C₁₂, corresponding to lauryl, in this case preferably n=0.

When mixtures are used, mixtures of compounds (I) wherein n=0 and the aliphatic chain ranges from C₁₀ to C₁₄ are preferably used.

The compositions of the invention can be in the form of toothpastes containing the zinc compounds of formula (I) in an amount (per cent by weight) from 0.8% to 7%, preferably from 1 to 5%, still more preferably from 1,5% to 3,5%.

The pH of the formulation is higher than or equal to 6, preferably comprised between 6 and 7.5.

The compositions of the invention are in the form of toothpastes or mouthwashes. The toothpaste components which can be added are those usually utilized in this kind of formulations. Humectants, preservatives, thickeners, dyes, aromas, sweeteners, abrasives and optionally detergents, vegetable extracts, pH correctors can be mentioned.

As humectant agents, water, polyalcohols and polyglycols can for example be mentioned. Glycerine, sorbitol, xylitol can for example be mentioned.

As preservative agents, agents releasing formaldehyde, paraoxybenzoic acid and its esters, etc. can be used. Parabens and imidazolidinylurea can for example be mentioned.

As thickening agents, cellulose derivatives, carboxyvinylpolymers, alginates, rubbers and carragenines can for example be mentioned. In particular esters of carboxymethyl cellulose or of hydroxyethylcellulose are used.

Dye agents that can be used are of both organic and inorganic type, such as pigments. Those of conventional cosmetic use, for example CI 47005, CI 42051, CI 45430 can be used, see Annexe 4, first part of the Italian Law 11 October 1986 713/86 and subsequent amendments.

The usable aromas are for example mint, clove, anise, orange, bergamot, eucalyptus derivatives, etc..

The sweeteners that can be used are those known in the art for applications in toothpaste formulations. Preferably sweeteners having a low caloric content and they are non cariogenic such as saccharin and derivatives thereof, xylitol, and mannitol, are used.

Abrasives can be selected for example from silicas, alumina, phosphates and carbonates, for example dihydrate calcium phosphate, calcium carbonate and precipitated silicas.

The optional detergents can be selected for example from non ionic, anionic, cationic and amphoteric surfactants such as for example sodium laurylsulphate, cocamidopropyl betaine, taurates, sulphosuccinates, etc.. As said, the compounds of the invention in their formulation do not require surfactants since they act themselves as surfactants.

As pH correctors, weak or strong acids or bases can be used. Citric acid, soda, sodium carbonate, triethanolamine, arginine, etc. can be mentioned.

When the compositions of the present invention are in the form of mouthwashes, they can contain ethyl alcohol and other excipients soluble in water or in hydroalcoholic solutions, such as humectants, surfactants, dyes, preservatives, aromas, vegetable extracts, pH correctors as above indicated.

The compositions of the invention when used in the mouthwash form, contain the zinc compounds of formula (I) in an amount, expressed as percent by weight, from 0.8% to 2%.

If desired, in the formulations according to the present invention other active principles of compositions for the oral hygiene can be added. For example fluorine compounds, preferably inorganic fluorine compounds, can be added. The following ones can for example be mentioned:
- alkaline and alkaline-earth metal fluorides, such as sodium fluoride, ammonium fluoride, calcium fluoride,
- alkaline and alkaline-earth metal monofluoro-phosphates, such as sodium monofluorophosphate, calcium monofluorophosphate, ammonium monofluorophosphate, magnesium monofluorophosphate,
- alkaline and alkaline-earth metal fluorosilicates, such as for example sodium fluorosilicate.

In the formulations of the present invention the amount of said compounds is comprised between 0.01 and 2% by weight as fluorine. Preferably fluorinated compounds in an amount expressed as % by weight of fluorine, from 0.02 to 0.15% are used.

To the formulations of the present invention imidoalkanpercarboxylic acids can also be added, having general formula: 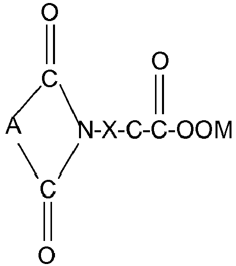 wherein:
A indicates a group selected from the following: 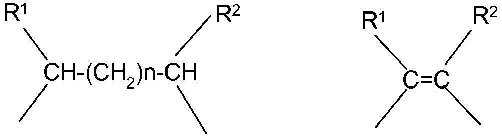 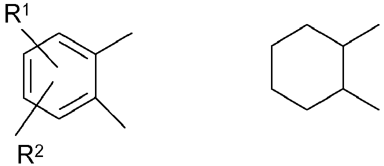 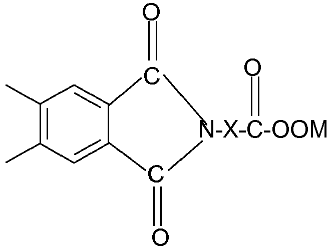
   n is an integer and is 0, 1 or 2,
   R¹ is selected from hydrogen, chlorine, bromine C₁-C₂₀ alkyl, C₂-C₂₀ alkenyl, aryl or alkylaryl,
   R² is selected from hydrogen, chlorine, bromine or a group of formula

      -SO₃M, -CO₂M, -CO₃M, -OSO₃M,
   M is selected from hydrogen, an alkaline metal or ammonium ion or the equivalent of an alkaline-earth metal ion,
   X is selected from C₁-C₁₉ alkylene or arylene,
   Y is C₃-C₁₉ alkylene.

Preferred imidoalkanpercarboxylic acids are phthalimido-peroxycarboxylic acids, more preferably epsilon-phthalimido-peroxyhexanoic acid, known as PAP.

Preferably the imidoalkanpercarboxylic acids are in the form of adducts with cyclodextrins, preferably beta cyclodextrins. These adducts are described in EP 1,050,294 in the name of the Applicant.
The molar ratio imidoalkanpercarboxylic acids/cyclodextrins in the imidoalkanpercarboxylic acid-cyclodextrin adduct is comprised between 1:1 and 1:2.
The amount of these adducts ranges from 0.05 to 10% by weight, preferably 0.1-7.5% by weight, of the formulation comprising the compounds of formula (I).

Among cyclodextrins, the following can be mentioned:
alpha, beta, gamma or delta cyclodextrins,
alpha, beta, gamma or delta cyclodextrins modified with acyl, alkyl, hydroxyalkyl groups, the alkyl having generally from 1 to 10 carbon atoms, preferably from 2 to 6.

Mixtures of said cyclodextrins and/or mixtures of said cyclodextrins with dextrins and/or with linear oligosaccharides can also be used.

Preferably beta-cyclodextrins are used.

A process for preparing the adducts of the imidoalkanpercarboxylic acids, in particular of epsilon-phthalimidoperoxyhexanoic acid, with cyclodextrins, in particolar beta cyclodextrins, is described in EP 895,777, herein incorporated by reference.

Unexpectedly and surprisingly the compounds of the invention of formula (I), in formulations for the oral cavity hygiene show on the bacterial strains of the oral cavity an activity equal to or comparable to that of Triclosan, compound commercially used.

Formula (I) compounds containing compositions show unexpectedly and surprisingly an high stability (see the examples). Further the compositions surprisingly and unexpectedly are perfectly compatible with the components of the formulations of toothpastes and mouthwashes for oral use of Triclosan, i.e., they are drop-in of the Triclosan used nowadays. See the stability tests reported in the examples.

In the stability tests, see the examples, it has been found that the formulations containing the compounds of formula (I) are as stable as those containing Triclosan.

Besides, the antibacterial activity in the oral cavity of the compounds of the present invention has a broad spectrum, since they are active towards Streptococci and towards the other microorganisms of the oral cavity flora: actinomycetes, staphylococci, as well as on yeasts (Candida Albicans).

As said, the use of zinc compounds of formula (I) in formulations for the oral cavity hygiene has surprisingly and unexpectedly shown a high antibacterial activity on the oral cavity flora combined with high stability of the formulations. Other zinc compounds with free zinc ions are preferably not used in the compositions of the invention. In fact it has been found that by adding these zinc compounds, the stability of the formulation is compromized. Furthermore the compounds of the invention can be used in tooth compositions without requiring the addition as essential component of surfactants. Of course, if desired, these can be used.

The following examples are reported for illustrative purposes.

### EXAMPLES

### Formulations

### Example 1

5 kg of a tooth formulation were prepared, having the following composition (% by weight):

| | |
|---|---|
| 27% w/w solution in water of lauryl zinc sulphate (INCI: Zinc Coco-sulphate) corresponding to 2.16% of the pure compound | 8.00 |
| Glycerine | 40.00 |
| Parabens | 0.25 |
| Sodium carboxymethyl cellulose | 1.50 |
| Imidazolidinyl urea | 0.25 |
| Sodium Saccharinate | 0.20 |
| Tetrasodium Pyrophosphate | 0.18 |
| Titanium Dioxide | 0.50 |
| Hydrated silica | 23.00 |
| Sodium monofluorophosphate | 0.50 |
| Sodium fluoride | 0.10 |
| Aroma | 1.80 |
| Sodium carbonate | 0.08 |
| Water the difference to 100% by weight | |
| pH | 6.8 |

The antibacterial activity of the composition has been evaluated in example 4.

### Example 2 comparative

5 kg of a tooth formulation were prepared, the composition being the following (% by weight):

| | |
|---|---|
| Triclosan | 0.10 |
| Lauryl sodium sulphate (99%) | 1.70 |
| Glycerine | 40.00 |
| Parabens | 0.25 |
| Sodium carboxymethyl cellulose | 1.50 |
| Imidazolidinyl urea | 0.25 |
| Sodium saccharinate | 0.20 |
| Tetrasodium Pyrophosphate | 0.18 |
| Titanium dioxide | 0.50 |
| Hydrated silica | 23.00 |
| Sodium monofluorophosphate | 0.50 |
| Sodium fluoride | 0.10 |
| Aroma | 1.80 |
| Water the difference to | 100 |
| pH | 6.8 |

The antibacterial activity of the composition has been evaluated in the example 3 comparative.

### Characterization of the above reported formulations

### Ex. 3 comparative

### Evaluation of the antibacterial activity "in vitro" of the toothpaste formulation of example 2 comparative containing Triclosan on the microorganism flora of the oral cavity.

### EXPERIMENTAL PROCEDURE

The microorganisms used in the test were the following:
- *Streptococcus mutans*
- *Streptococcus agalactiae*
- *Staphylococcus aureus*
- *Actinomycetes*
- *Candida albicans (YEAST)*

### Culture medium:

- Tryptone Soya Broth (TSB), for the preparation of the suspensions of the standard microorganism strains
- Tryptic Soy Agar (ready made culture medium in Petri plates).

The contact time for each sample of the formulation under examination and for each of the microorganism strains used was of 48 h at a constant temperature of + 37°C.

The activity of the formulation has been assayed by agar plate diffusion. The method allows to quantitatively evaluate the sensitivity of different microorganism strains towards the tested toothpaste compositions. The effectiveness of the compositions in inhibiting the microorganism growth (bacterial or micotic) in Tryptic Soy Agar medium was evaluated on the basis of the formation and size of the inhibitory halos of microorganism growth. The formation of an halo reveals the inhibitory activity of the toothpaste compositions against the microorganisms under examination.

Nitrocellulose discs having 1,0 cm diameter were contacted for 4 hours with an aqueous solution comprising mucin, proteins and sugar, i.e. the same components of human saliva. They were then dried at room temperature. The so treated discs were dipped for 30 min. into an homogeneous suspension containing 10% w/w of the toothpaste of example 2 comparative. The discs were then rinsed in sterile water, dried at room temperature, and transferred on ready made plates of Tryptic Soy Agar culture medium. The medium has been previously inoculated with the bacterial species under examination.

After an incubation of 48 hours at 37°C, the bacterial growth inhibition was evaluated by measuring the area of the inhibition zone (halo), taking into account also the bacterial growth occurred under the disc.

More in detail, the results were evaluated on the basis of the formed halo diameter and on this basis halos were rated according to the following score:
An halo of great size was scored +++.

The antibacterial activity was indicated in the Table with S, that is the microorganism strain was found sensitive to the toothpaste formulation under test.
An halo of average size was scored ++.

The antibacterial activity was indicated in the Table with M, that is the microorganism strain was found on the average sensitive to the toothpaste formulation.
An halo of small size was scored +,
that is the microorganism strain was found slightly sensitive to the tested toothpaste formulation.

The antibacterial activity was indicated in the Table with L.
The absence of an halo is indicated with -.

In this case the microorganism strain was not sensitive (resistant) to the toothpaste formulation. The absence of activity was indicated with R.

The results are reported in Table 1.

**Table 1**

| Microorganism growth inhibition of the toothpaste of ex. 2 comparative containing Triclosan | | | | |
|---|---|---|---|---|
| Microrganisms | Microbial concentration | Halo size (diameter - cm) | Halo size score | Microbial strain sensitivity |
| Streptococcus mutans | 10⁶ | 3.5 | +++ | S |
| Streptococcus agalactiae | 10⁶ | 3.5 | +++ | S |
| Staphylococcus Aureus | 10⁶ | 3.5 | +++ | S |
| Actinomycetes | 10⁶ | 3.0 | +++ | S |
| Candida Albicans | 10⁶ | 2.7 | +++ | S |

### Ex. 4

### Evaluation of the antibacterial activity "in vitro" of the toothpaste formulation of ex. 1 containing the compound of formula (I) on the flora of the oral cavity.

The experimental procedure reported in Ex. 3 comparative was repeated but using the formulation of ex. 1.

The results of microorganism growth inhibition are reported in Table 2.

**Table 2**

| Microorganism growth inhibition of the toothpaste of ex. 1 containing 2.16% of zinc lauryl sulphate | | | | |
|---|---|---|---|---|
| Microrganisms | Microbial Concentration | Halo size (diameter - cm) | Halo size score | Microbial strain sensitivity |
| Streptococcus mutans | 10⁶ | 2.8 | +++ | S |
| Streptococcus agalactiae | 10⁶ | 2.6 | +++ | S |
| Staphylococcus Aureus | 10⁶ | 3.0 | +++ | S |
| Actinomycetes | 10⁶ | 2.5 | +++ | S |
| Candida Albicans | 10⁶ | 2.6 | +++ | S |

By comparing the results reported in Tables 1 and 2, it is noticed that at the tested doses the toothpaste of the invention containing zinc laurylsulphate is as active as Triclosan in inhibiting the growth of all the tested microorganisms.

### Formulation

### Ex. 5 comparative

5 kg of a toothpaste formulation was prepared, having the following composition (% by weight):

| | |
|---|---|
| 27% w/w solution in water of zinc lauryl sulphate (INCI: Zinc Coco-sulphate), corresponding to 0.4% of the pure compound | 1.50 |
| Glycerine | 40.00 |
| Parabens | 0.25 |
| Sodium carboxymethyl cellulose | 1.50 |
| Imidazolidinyl urea | 0.25 |
| Sodium Saccharinate | 0.25 |
| Tetrasodium Pyrophosphate | 0.18 |
| Titanium dioxide | 0.50 |
| Hydrated silica | 23.00 |
| Sodium monofluorophosphate | 0.50 |
| Sodium fluoride | 0.10 |
| Aroma | 1.80 |
| Sodium carbonate | 0.08 |
| Water the difference to 100% by weight. | |
| pH | 6.8 |

The antibacterial activity of the composition has been evaluated in example 6 comparative.

### Characterization

### Ex. 6 comparative

### Evaluation of the antibacterial activity "in vitro" of the toothpaste of ex. 5 comparative on the flora of the oral cavity

The experimental procedure reported in ex. 3 comparative was repeated but with the formulation of ex. 5 comparative.

The inhibitory test results for microorganism growth are reported in Table 3.

**Table 3**

| Microorganism growth inhibition of the toothpaste of ex. 1 containing zinc laurylsulphate in an amount equal to 0.4% by weight | | | | |
|---|---|---|---|---|
| Microrganisms | Microbial concentration | Halo size (diameter - cm) | Halo size score | Microbial strain sensitivity |
| Streptococcus mutans | 10⁶ | None | - | R |
| Streptococcus agalactiae | 10⁶ | " | - | R |
| Staphylococcus Aureus | 10⁶ | 1.5 | - | R |
| Actinomycetes | 10⁶ | None | - | R |
| Candida Albicans | 10⁶ | " | - | R |

The Table shows that the formulation containing zinc lauryl sulphate of example 5 comparative cannot be used as substitute of Triclosan formulation for the hygiene of the oral cavity as it does not show any antibacterial activity.

### Formulation

### Ex. 7

### Mouthwash formulation

5 Kg of a mouthwash formulation, having the following composition (% by weight) were prepared

| | |
|---|---|
| 27% w/w solution in water of zinc lauryl sulphate (INCI: Zinc Coco-sulphate), corresponding to 2.16% of the pure compound | 8.00 |
| Propylene glycol | 3.00 |
| Sodium benzoate | 0.50 |
| Sodium bicarbonate | 0.15 |
| Sodium fluoride | 0.06 |
| Glycol extract of Asiatic Centella | 0.15 |
| Sodium Saccharinate | 0.02 |
| 2-bromo-2-nitropropan-1,3-diol | 0.05 |
| Lactic acid | 0.21 |
| Sodium lactate | 0.21 |
| 40 mole ethoxylated hydrogenated castor oil | 1.00 |
| Polysorbate 20 | 1.20 |
| Aroma | 0.30 |
| Dye CI 42051 | 0.0008 |
| Dye CI 47005 | 0.0008 |
| Water the difference to 100% by weight. | |
| pH | 6.05 |

### Characterization

### Ex. 8

### Stability tests with the tooth composition of example 1

From a preparation of 5 kg of toothpaste of ex. 1, 6x200 ml samples were taken and each transferred into a 220 ml beaker that was then hermetically sealed.

At time zero the following parameters were determined:
- appearance
- colour
- taste
- pH of the toothpaste as such, determined at 20C.

The samples were conditioned at the times indicated in the Table and at the following temperatures: 5°C, 25°C, 40°C. Two samples for each temperature were conditioned.

The results are reported in Table 4.

### Ex. 9 comparative

### Stability tests with the toothpaste of ex. 2 comparative

The stability tests were carried out as in Ex. 8. The results are reported in Table 5. It is noticed that the data of this Table are the same as those reported in Table 4.

Therefore the composition of the toothpaste of example 1 is stable as the preparation that contains Triclosan. This shows that the compounds of formula (I) have the same compatibility of Triclosan with the additives used for these preparations.

### Ex. 10

### Stability tests of the mouthwash of ex. 7

From 5 litres of mouthwash having the composition of ex. 7, 6x200 ml samples were taken and each transferred into a 220 ml beaker with an hermetic seal. Stability tests were carried out as in example 8.

The results are reported in Table 6.

### Formulation

### Ex. 11

5 kg of toothpaste containing the compounds of the present invention and the adduct PAP-cyclodextrins were prepared. The composition was as it follows (% by weight)

| | |
|---|---|
| 27% w/w solution in water of zinc lauryl sulphate INCI: Zinc Coco-sulphate), corresponding to 2.16% of the pure compound | 8.00 |
| PAP-cyclodextrin adduct | 6.50 |
| Sorbitol | 50.00 |
| Xylitol | 5.00 |
| Sodium carboxymethyl cellulose | 1.50 |
| Imidazolidinyl urea | 0.15 |
| Sodium saccharinate | 0.14 |
| Polysorbate 60 | 4.00 |
| Encapsulated citric acid | 0.60 |
| Jojoba and dye esters | 0.20 |
| Titanium dioxide | 0.40 |
| Hydrated silica | 24.00 |
| Sodium fluoride | 0.32 |
| Aroma | 1.50 |
| Water enough to | 100 |

Stability tests have been carried out by the Applicant on the toothpaste composition of this example by using the same protocol as in example 8. It was noticed that the composition of example 11 resulted stable.

**Table 4**

| Stability tests at 5°C, 25°C and 40°C of the toothpaste of ex. 1 containing 2.16% of zinc laurylsulphate | | | | | | | | |
|---|---|---|---|---|---|---|---|---|
| T (°C) | Time zero | | | | After 6 months | | | |
| | Appearance | Colour | Taste* | PH | Appearance | Colour | Taste* | pH |
| 5 | Glossy viscous paste | white | Unchanged | 6.8 | Glossy viscous paste | white | Unchanged | 6.8 |
| 25 | Glossy viscous paste | white | Unchanged | 6.8 | Glossy viscous paste | white | Unchanged | 6.75 |
| 40 | Glossy viscous paste | white | Unchanged | 6.8 | Glossy viscous paste | white | Unchanged | 6.60 |

| | | | | | | | | |
|---|---|---|---|---|---|---|---|---|
| * With respect to the laboratory standard | | | | | | | | |

**Table 5**

| Stability tests at 5°C, 25°C and 40°C of the toothpaste of ex. 2 Comparison containing Triclosan | | | | | | | | |
|---|---|---|---|---|---|---|---|---|
| T (°C) | Time zero | | | | After 6 months | | | |
| | Appearance | Colour | Taste* | PH | Appearance | Colour | Taste* | PH |
| 5 | Glossy viscous paste | White | Unchanged | 6,8 | Gllossy viscous paste | White | Unchanged | 6.8 |
| 25 | Glossy viscous paste | White | Unchanged | 6,8 | Glossy viscous paste | White | Unchanged | 6.75 |
| 40 | Glossy viscous paste | white | Unchanged | 6,8 | Glossy viscous paste | white | Unchanged | 6.60 |

| | | | | | | | | |
|---|---|---|---|---|---|---|---|---|
| * With respect to the laboratory standard | | | | | | | | |

**Table 6**

| Stability tests at 5°C, 25°C and 40°C of the mouthwash of ex. 7 containing 0.20% of zinc laurylsulphate | | | | | | | | |
|---|---|---|---|---|---|---|---|---|
| T (°C) | Time zero | | | | After 6 months | | | |
| | Appearance | Colour | Taste* | PH | Appearance | Colour | Taste* | pH |
| 5 | Limpid solution | green | Unchanged | 6.05 | Limpid solution | green | Unchanged | 6.2 |
| 25 | Limpid solution | green | Unchanged | 6.05 | Limpid solution | Green | Unchanged | 6.2 |
| 40 | Limpid solution | green | Unchanged | 6.05 | Limpid solution | Green | Unchanged | 6.2 |

| | | | | | | | | |
|---|---|---|---|---|---|---|---|---|
| * with respect to the laboratory standard | | | | | | | | |

## Claims

1. Use of zinc compounds having formula:
[R-(OCH₂CH₂)ₙ-OSO₃⁻] • 1/2 Zn²⁺ (I)
wherein R is a linear or branched saturated or unsaturated C₆-C₂₀ aliphatic chain,
n is an integer in the range from zero to 20, for the preparation of formulations as antibacterial agents of the oral cavity,
wherein the formulations are in the form of toothpastes and mouthwashes,
wherein the amount, in percent by weight, of the compounds of formula (I) in toothpastes ranges from 0.8% to 7% and in mouthwashes from 0.8% to 2%.

2. Use according to claim 1, wherein in formula (I) n=0.

3. Use according to claims 1-2, wherein mixtures of the compounds of formula (I) are used.

4. Use according to claims 1-3 wherein in the compounds of formula (I) the aliphatic chain is a C₈-C₁₈ linear aliphatic chain.

5. Use according to claims 1-4 wherein in the compounds of formula (I) the aliphatic chain is a C₁₂ linear aliphatic chain and n=0.

6. Use according to claim 3 wherein mixtures of the compounds of formula (I) with n=0 and the aliphatic chain is a C₁₀ to C₁₄ aliphatic chain.

7. Use according to claims 1-6 wherein the pH of the formulations is higher than or equal to 6.

8. Use according to claims 1-7 wherein the formulations further comprise fluorine compounds, in an amount from 0.01 to 2% by weight as fluorine.

9. Use according to claims 1-8, wherein the formulations further comprise imidoalkanpercarboxylic acids of general formula 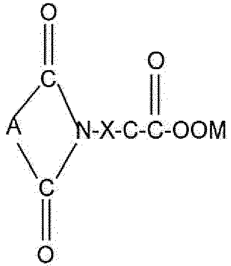 wherein:
A indicates a group selected from the following formulas: 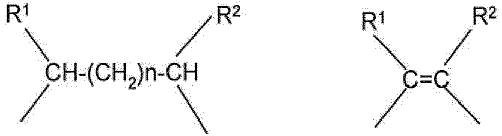 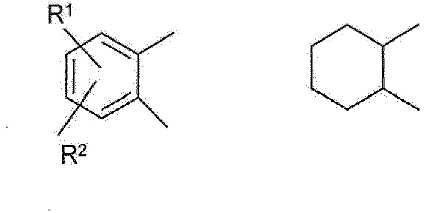 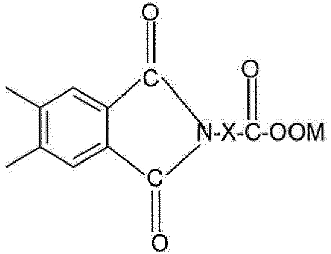
n is an integer selected from 0, 1 or 2,
R¹ is selected from hydrogen, chlorine, bromine, C₁-C₂₀ alkyl, C₂-C₂₀ alkenyl, aryl or alkylaryl,
R² is selected from hydrogen, chlorine, bromine or a group of formula
-SO₃M, -CO₂M, -CO₃M, -OSO₃M,
M is selected from hydrogen, an alkaline metal or ammonium ion or the equivalent of an alkaline-earth metal ion,
X is selected from C₁-C₁₉ alkylene or arylene;
Y is a C₃-C₁₉ alkylene.

10. Use according to claim 9 wherein the imidoalkanpercarboxylic acid is epsilon-phthalimidoperoxyhexanoic acid.

11. Use according to claims 9-10 wherein the imidoalkanpercarboxylic acids are in the form of adducts with cyclodextrins, the molar ratio imidoalkanpercarboxylic acids/cyclodextrins being in the range 1:1 - 1:2.

12. Use according to claim 11 wherein the imidoalkanpercarboxylic acid/cyclodextrins adduct is the adduct between epsilon--phthalimido peroxyhexanoic acid and beta cyclodextrins.

13. Use according to claims 1-12 wherein the amount of the adducts of the imidoalkanpercarboxylic acids in the formulations of the compounds of formula (I) ranges from 0.05 to 10% by weight.

## Patentansprüche

1. Verwendung von Zinkverbindungen der Formel:
[R-(OCH₂CH₂)ₙ-OSO₃⁻] • ½ Zn²⁺ (I)
worin R eine geradkettige oder verzweigte gesättigte oder ungesättigte aliphatische C₆-C₂₀-Kette ist,
n eine ganze Zahl im Bereich von 0 bis 20 ist,
zur Herstellung von Formulierungen zur Verwendung als antibakterielle Mittel für die Mundhöhle,
wobei die Formulierungen in Form von Zahnpasten und Mundwässern vorliegen,
wobei die Menge der Verbindungen der Formel (I), in Gewichtsprozent, in Zahnpasten im Bereich von 0,1 % bis 7 % und in Mundwässern von 0,8 % bis 2 % liegt.

2. Verwendung nach Anspruch 1, wobei in Formel (I) n=0.

3. Verwendung nach den Ansprüchen 1-2, wobei Mischungen der Verbindungen der Formel (I) verwendet werden.

4. Verwendung nach den Ansprüchen 1-3, wobei die aliphatische Kette in den Verbindungen der Formel (I) eine geradkettige aliphatische C₈-C₁₈-Kette ist.

5. Verwendung nach den Ansprüchen 1-4, wobei die aliphatische Kette in den Verbindungen der Formel (I) eine geradkettige aliphatische C₁₂-Kette ist.

6. Verwendung nach Anspruch 3, wobei Mischungen der Verbindungen der Formel (I) mit n=0 verwendet werden und die aliphatische Kette eine geradkettige aliphatische C₁₀-C₁₄-Kette ist.

7. Verwendung nach den Ansprüchen 1-6, wobei der pH der Formulierungen höher als oder gleich 6 ist.

8. Verwendung nach den Ansprüchen 1-7, wobei die Formulierungen der Verbindungen der Formel (I) Fluorverbindungen in einer Menge von 0,01 bis 2 Gew.-% als Fluor umfassen.

9. Verwendung nach den Ansprüchen 1-8, wobei die Formulierungen der Verbindungen der Formel (I) Imidoalkanpersäuren der allgemeinen Formel 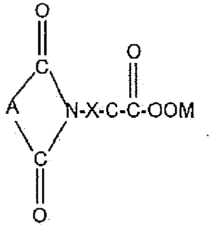 umfassen, worin:
A eine Gruppe bedeutet, die ausgewählt ist aus den folgenden Formeln: 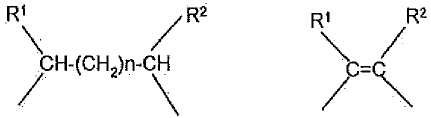 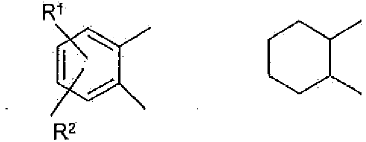 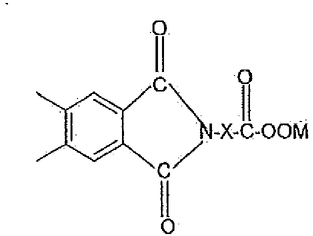
n eine ganze Zahl ist, die ausgewählt ist aus 0, 1 oder 2, R¹ ausgewählt ist aus Wasserstoff, Chlor, Brom, C₁-C₂₀-Alkyl, C₂-C₂₀-Alkenyl, Aryl oder Alkylaryl,
R² ausgewählt ist aus Wasserstoff, Chlor, Brom oder einer Gruppe der Formel -SO₃M, -CO₂M, -CO₃M, -OSO₃M,
M ausgewählt ist aus Wasserstoff, einem Alkalimetall- oder Ammonium-Ion oder dem Äquivalent eines Erdalkalimetall-Ions, X ausgewählt ist aus C₁-C₁₉-Alkylen oder Arylen;
Y ein C₃-C₁₉-Alkylen ist.

10. Verwendung nach Anspruch 9, wobei die Imidoalkanpersäure epsilon-Phthalimidoperoxyhexansäure ist.

11. Verwendung nach den Ansprüchen 9-10, wobei die Imidoalkanpersäuren in Form von Addukten mit Cyclodextrinen vorliegen, wobei das Molverhältnis Imidoalkanpersäuren/Cyclodextrine im Bereich von 1:1 - 1:2 liegt.

12. Verwendung nach Anspruch 11, wobei das Imidoalkanpersäure/ Cyclodextrin--Addukt das Addukt zwischen epsilonPhthalimidoperoxyhexansäure und beta-Cyclodextrinen ist.

13. Verwendung nach den Ansprüchen 1-12, wobei die Menge des Addukts der Imidoalkanpersäuren in den Formulierungen der Verbindungen der Formel (I) im Bereich von 0,05 bis 10 Ges.-% liegt.

## Revendications

1. Utilisation de composés de zinc de formule :
[R- (OCH₂CH₂)ₙ-OSO₃⁻] • 1/2 Zn²⁺ (I)
dans laquelle R représente une chaîne aliphatique en C₆ à C₂₀ saturée ou non saturée, linéaire ou ramifiée,
n représente un nombre entier dans la plage de zéro à 20,
pour la préparation de formulations comme des agents antibactériens de la cavité buccale,
dans laquelle les formulations sont sous forme de dentifrices et de bains de bouche,
dans laquelle la quantité, en pourcentage en poids, des composés de formule (I) dans les dentifrices se situe dans la plage de 0,8 % à 7 % et dans les bains de bouche, de 0,8 % à 2 %.

2. Utilisation selon la revendication 1, dans laquelle, dans la formule (I), n = 0.

3. Utilisation selon les revendications 1 ou 2, dans laquelle des mélanges des composés de formule (I) sont utilisés.

4. Utilisation selon les revendications 1 à 3, dans laquelle, dans les composés de formule (I), la chaîne aliphatique est une chaîne aliphatique linéaire en C₈ à C₁₈.

5. Utilisation selon les revendications 1 à 4, dans laquelle, dans les composés de formule (I), la chaîne aliphatique est une chaîne aliphatique linéaire en C₁₂ et n = 0.

6. Utilisation selon la revendication 3, dans laquelle, dans les mélanges des composés de formule (I), n = 0 et la chaîne aliphatique est une chaîne aliphatique en C₁₀ à C₁₄.

7. Utilisation selon les revendications 1 à 6, dans laquelle le pH des formulations est supérieur ou égal à 6.

8. Utilisation selon les revendications 1 à 7, dans laquelle les formulations comprennent en outre des composés de fluor, dans une quantité de 0,01 à 2 % en poids en tant que fluor.

9. Utilisation selon les revendications 1 à 8, dans laquelle les formulations comprennent en outre des acides imido-alcane-percarboxyliques de formule générale : 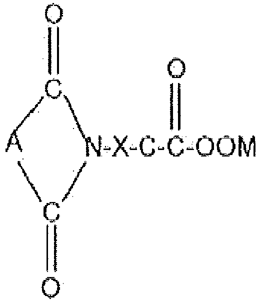 dans laquelle :
A indique un groupe choisi parmi les formules suivantes : 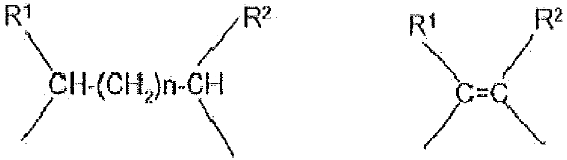 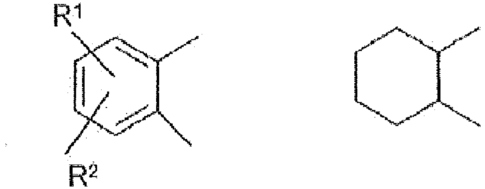 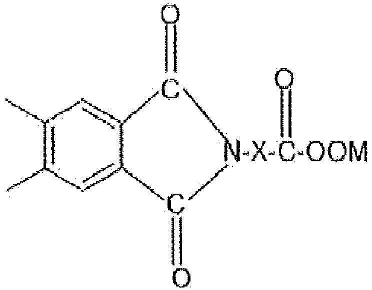
n représente un nombre entier choisi parmi 0, 1 ou 2,
R¹ est choisi parmi un atome d'hydrogène, de chlore, de brome, un groupe alkyle en C₁ à C₂₀, alcényle en C₂ à C₂₀, aryle ou alkylaryle,
R² est choisi parmi un atome d'hydrogène, de chlore, de brome ou un groupe de formule :
-SO₃M, -CO₂M, -CO₃M, -OSO₃M,
M est choisi parmi un atome d'hydrogène, un métal alcalin ou un ion ammonium ou l'équivalent d'un ion de métal alcalinoterreux,
X est choisi parmi un groupe alkylène en C₁ à C₁₉ ou arylène ;
Y représente un groupe alkylène en C₃ à C₁₉.

10. Utilisation selon la revendication 9, dans laquelle l'acide imido-alcane-percarboxylique est l'acide epsilon-phtalimido-peroxyhexanoïque.

11. Utilisation selon la revendications 9 ou 10, dans laquelle les acides imido-alcane-percarboxyliques sont sous forme de produits d'addition avec des cyclodextrines, le rapport molaire des acides imido-alcane-percarboxyliques/cyclodextrines se situant dans la plage de 1/1 à 1/2.

12. Utilisation selon la revendication 11, dans laquelle le produit d'addition d'acide imido-alcane-percarboxylique/cyclodextrine est le produit d'addition entre l'acide epsilon-phtalimido-peroxyhexanoïque et des bêta-cyclodextrines.

13. Utilisation selon les revendications 1 à 12, dans laquelle la quantité des produits d'addition des acides imido-alcane-percarboxyliques dans les formulations des composés de formule (I) se situe dans la plage de 0,05 à 10 % en poids.
